(19) Europäisches Patentamt
European Patent Office
Office européen des brevets

(11) **EP 3 192 826 A1**

(12) **EUROPEAN PATENT APPLICATION**
published in accordance with Art. 153(4) EPC

(43) Date of publication:
**19.07.2017 Bulletin 2017/29**

(51) Int Cl.:
*C08J 3/24* (2006.01)          *C08J 3/075* (2006.01)
*C08F 2/10* (2006.01)          *C08J 9/10* (2006.01)
*C08J 9/14* (2006.01)

(21) Application number: **15900610.5**

(22) Date of filing: **14.12.2015**

(86) International application number:
**PCT/KR2015/013676**

(87) International publication number:
**WO 2017/086528 (26.05.2017 Gazette 2017/21)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **17.11.2015 KR 20150161157**

(71) Applicant: **LG Chem, Ltd.**
**Seoul 07336 (KR)**

(72) Inventors:
• **HEO, Seong Beom**
**Daejeon 34122 (KR)**

• **JANG, Tae Hwan**
**Daejeon 34122 (KR)**
• **KIM, Mi Young**
**Daejeon 34122 (KR)**
• **JEONG, Jiyoon**
**Daejeon 34122 (KR)**
• **KIM, Bhom Ri**
**Daejeon 34122 (KR)**

(74) Representative: **Goddar, Heinz J.**
**Boehmert & Boehmert**
**Anwaltspartnerschaft mbB**
**Pettenkoferstrasse 22**
**80336 München (DE)**

(54) **METHOD FOR PRODUCING SUPERABSORBENT RESIN**

(57)    The preparation method of superabsorbent polymer according to the present invention can increase suction power without degradation of other properties of superabsorbent polymer, and thus, the prepared superabsorbent polymer may be usefully used as material of hygienic goods such as a diaper.

[FIG. 2]

**Description**

**[CROSS-REFERENCE TO RELATED APPLICATION(S)]**

This application claims the benefit of Korean Patent Application No. 10-2015-0161157 filed on November 17, 2015 with the Korean Intellectual Property Office, the disclosures of which are herein incorporated by reference in their entirety.

**[TECHNICAL FIELD]**

The present invention relates to a method for preparing superabsorbent polymer having excellent suction power.

**[BACKGROUND OF ART]**

Super absorbent polymer (SAP) is synthetic polymer material that can absorb moisture of 500 to 1000 times of self-weight, and is also named as super absorbency material (SAM), absorbent gel material (AGM), and so on. The superabsorbent polymer began to be commercialized as sanitary items, and currently, it is being widely used as hygienic goods such as a disposable diaper and so on, water-holding material for soil, water stop material for civil engineering and architecture, sheets for raising seedling, freshness preservatives in the field of food circulation, and so on.

As a method for preparing the superabsorbent polymer, a reverse phase suspension polymerization method or an aqueous polymerization method, and the like are known. The reverse phase suspension polymerization method is disclosed in, for example, Japanese Patent Laid-Open Publication No. Sho 56-161408, Japanese Patent Laid-Open Publication No. Sho 57-158209, and Japanese Patent Laid-Open Publication No. Sho 57-198714, and so on. Further, as the aqueous polymerization method, a thermal polymerization method wherein hydrous gel phase polymer is polymerized while breaking and cooling in a kneader equipped with several shafts, and a photo-polymerization method wherein an aqueous solution of high concentration is simultaneously polymerized and dried by irradiating UV on a belt, and the like are known.

Meanwhile, important performances of hygienic goods such as a diaper, and so on, include intake time and wet back. With gradual thinning of diapers, the importance of permeability is increasing so as to enable efficient diffusion of material absorbed into superabsorbent polymer used therein, and in order to improve dryness of a diaper, it is important to improve suction power capable of drawing urine from the pulp.

In order to increase suction power, the internal cross-linking degree of superabsorbent polymer should be lowered and the content of silica or inorganic particles on the surface should be reduced. However, in this case, degradation of properties other than suction power, for example, absorbency under load or gel bed permeability, is inevitable. Thus, there is an urgent demand for a preparation method that can increase suction power without degradation of other properties of superabsorbent polymer.

**[DETAILED DESCRIPTION OF THE INVENTION]**

**[Technical Problem]**

It is an object of the present invention to provide a method for preparing superabsorbent polymer having excellent suction power.

**[Technical Solution]**

In order to achieve the object, the present invention provides a method for preparing superabsorbent polymer comprising the steps of

1) polymerizing and cross-linking a monomer composition comprising a water-soluble ethylene-based unsaturated monomer, a polymerization initiator, a first cross-linking agent and a blowing agent to form a hydrous gel phase polymer,
2) drying the hydrous gel phase polymer,
3) grinding the dried polymer, and
4) carrying out a surface cross-linking reaction by reacting the ground polymer at 170 to 250°C for 50 minutes or more in the presence of a second cross-linking agent.

A speed at which superabsorbent polymer absorbs moisture is referred to as 'suction power', which may be evaluated as the amount of saline water absorbed by 1g of superabsorbent polymer for 5 minutes, as described below. In hygienic goods such as a diaper, urine is first absorbed into pulp, and then, absorbed from the pulp to superabsorbent polymer. Thus, since superabsorbent polymer should absorb urine from pulp within a short time so that the wear sensation of a user may increase, a suction power is an important property of superabsorbent polymer.

However, in order to increase suction power, the internal cross-linking degree of superabsorbent polymer should be lowered and the content of silica or inorganic particles on the surface should be reduced, which causes degradation of other properties of superabsorbent polymer, for example, absorbency under load or gel bed permeability.

The present invention is characterized in that, as described below, by using a blowing agent in the process of forming, and using a high temperature condition when surface cross-linking hydrous gel phase polymer, suction power may be increased without degradation of other properties of superabsorbent polymer.

Hereinafter, the present invention will be explained in detail according to each step.

**Step of forming a hydrous gel phase polymer from a water-soluble ethylene-based unsaturated monomer (step 1)**

First, the preparation method of superabsorbent polymer comprises a step of forming a hydrous gel phase polymer from a water-soluble ethylene-based unsaturated monomer.

The water-soluble ethylene-based unsaturated monomer included in the monomer composition may be any monomer commonly used in the preparation of superabsorbent polymer. As a non-limiting example, the water-soluble ethylene-based unsaturated monomer may be a compound represented by the following Chemical Formula 1:

$$[\text{Chemical Formula 1}] \qquad R_1\text{-COOM}^1$$

in the Chemical Formula 1,
$R_1$ is a $C_{2-5}$ alkyl group comprising an unsaturated bond, and
$M^1$ is a hydrogen atom, a monovalent or divalent metal, an ammonium group, or an organic amine salt.

Preferably, the water-soluble ethylene-based unsaturated monomer may be one or more kind selected from the group consisting of acrylic acid, methacrylic acid, and monovalent metal salts, a divalent metal salts, ammonium salts and organic amine salts thereof. As such, if acrylic acid or a salt thereof is used as the water-soluble ethylene-based unsaturated monomer, superabsorbent polymer with improved absorption property may be obtained.

Besides, as the water-soluble ethylene-based unsaturated monomer, maleic anhydride, fumaric acid, crotonic acid, itaconic acid, 2-acryloylethane sulfonic acid, 2-methacryloylethane sulfonic acid, 2-(meth)acryloylpropane sulfonic acid or 2-(meth)acrylamide-2-methyl propane sulfonic acid, (meth)acrylamide, N-substituted (meth)acrylate, 2-hydroxyethyl (meth)acrylate, 2-hydroxypropyl (meth)acrylate, methoxypolyethyleneglycol (meth)acrylate, polyethylene glycol (meth)acrylate, (N,N)-dimethylaminoethyl (meth)acrylate, (N,N)-dimethylaminopropyl (meth)acrylamide, and so on, may be used.

Here, the water-soluble ethylene-based unsaturated monomer may have an acidic group, of which at least a part may be neutralized. Preferably, the monomers partially neutralized with alkali material such as sodium hydroxide, potassium hydroxide, ammonium hydroxide, and so on, may be used.

Wherein, the polymerization degree of the water-soluble ethylene-based unsaturated monomers may be 40 to 95 mol%, or 40 to 80 mol%, or 45 to 75 mol%. Although the range of the polymerization degree may vary according to the final properties, if the polymerization degree is too high, neutralized monomers may be precipitated, thus rendering it difficult to smoothly progress the polymerization, and to the contrary, if the polymerization degree is too low, the absorption power of the polymer may be significantly lowered, and elastic rubber-like properties may be exhibited, which is difficult to handle.

Further, the concentration of the water-soluble ethylene-based unsaturated monomer in the monomer composition may be appropriately controlled considering the polymerization time and reaction conditions, and so on, and preferably, it may be 20 to 90 wt%, or 40 to 65 wt%. Such a concentration range may be advantageous for controlling of grinding efficiency when grinding polymer as described below, without a need to remove non-reacted monomers after polymerizing using a gel effect appeared in the polymerization reaction of an aqueous solution of high concentration. However, if the concentration of monomers becomes too low, the yield of superabsorbent polymer may decrease. To the contrary, if the concentration of monomers becomes too high, process problems may arise such as precipitation of a part of monomers or decrease in grinding efficiency when grinding polymerized hydrous gel phase polymer, and so on.

Meanwhile, in the monomer composition, polymerization initiators commonly used in the preparation of superabsorbent polymer may be included. As non-limiting examples, as the polymerization initiators, a thermal polymerization initiator or a photo-polymerization initiator, and so on, may be used according to polymerization methods. However, even in the case of photo-polymerization, since a certain amount of heat is generated by UV irradiation, and so on, and heat is generated to some degree according to the progression of an exothermic polymerization reaction, a thermal polymerization initiator may be additionally included.

As the photo-polymerization initiator, for example, one or more compounds selected from the group consisting of benzoin ether, dialkyl acetophenone, hydroxyl alkylketone, phenyl glyoxylate, benzyl dimethyl ketal, acyl phosphine, and α-aminoketone may be used. As the specific example of the acyl phosphine, commercially used lucirin TPO, namely, 2,4,6-trimethyl-benzoyl-trimethyl phosphine oxide may be used. More various photo-polymerization initiators are described in Reinhold Schwalm, "UV Coatings: Basics, Recent Developments and New Application(Elsevier 2007)", page 115, which may be referred to.

Further, as the thermal polymerization initiator, at least one selected from the group consisting of a persulfate initiator, an azo initiator, hydrogen peroxide, and ascorbic acid may be used. Specific examples of the persulfate initiator may include sodium persulfate ($Na_2S_2O_8$) potassium persulfate ($K_2S_2O_8$) ammonium persulfate (($NH_4)_2S_2O_8$) and so on. Further, specific examples of the azo initiator may include 2,2-azobis(2-amidinopropane)dihydrochloride, 2,2-azo-bis-(N,N-dimethylene)isobutyramidine dihydrochloride, 2-(carbamoylazo)isobutyronitril, 2,2-azobis[2-(2-imidazolin-2-yl)propane]dihydrochloride, 4,4-azobis-(4-cyanovalericacid), and so on. More various thermal initiators are described in "Principle of Polymerization (Wiley, 1981)", Odian, page 203, which may be referred to.

The polymerization initiator may be added in the concentration of about 0.001 to 1 wt%, based on the monomer composition. That is, if the concentration of the polymerization initiator is too low, polymerization speed may become slow, and residual monomers may be extracted in large quantities in the final product. To the contrary, if the concentration of the polymerization initiator is too high, a polymer chain making up a network may become short, and thus, the properties of polymer may be degraded such as increase in the content of water soluble components and decrease in absorbency under load, and so on.

Meanwhile, the monomer composition comprises a first cross-linking agent (internal cross-linking agent) for improving the properties of polymer by the polymerization of the water-soluble ethylene-based unsaturated monomer. The initiators is intended to internally cross-link hydrous gel phase polymer, and is distinguished from the cross-linking agent (surface cross-linking agent) for cross-linking the surface of the hydrous gel phase polymer.

The internal cross-linking agent is not specifically limited as long as it enables the introduction of cross-link when polymerizing the water-soluble ethylene-based unsaturated monomer. As non-limiting examples, as the internal cross-linking agent, multifunctional cross-linking agents such as N,N'-methylenebisacrylamide, trimethylolpropane tri(meth)acrylate, ethylene glycol di(meth)acrylate, polyethylene glycol (meth)acrylate, propylene glycol di(meth)acrylate, polypropylene glycol (meth)acrylate, butanediol di(meth)acrylate, butylene glycol di(meth)acrylate, diethylene glycol di(meth)acrylate, hexanediol di(meth)acrylate, triethylene glycol di(meth)acrylate, tripropylene glycol di(meth)acrylate, tetraethylene glycol di(meth)acrylate, dipentaetythritol pentaacrylate, glycerin tri(meth)acrylate, pentaerythritol tetraacrylate, triarylamine, ethylene glycol diglycidyl ether, propylene glycol, glycerin, or ethylene carbonate may be used alone or in combinations of two or more kinds thereof, but not limited thereto.

The internal cross-linking agent may be added in the concentration of about 0.001 to 1 wt%, based on the monomer composition. That is, if the concentration of the internal cross-linking agent is too low, the absorption speed of superabsorbent polymer may decrease and gel strength may weaken. To the contrary, if the concentration of the internal cross-linking agent is too high, the absorption power of superabsorbent polymer may decrease, which may be not preferable as an absorber.

Meanwhile, the monomer composition also comprises a blowing agent for improving the suction power of superabsorbent polymer. The blowing agent generates gas in the process of forming hydrous gel phase polymer to expand the hydrous gel phase polymer, and thereby, micropores may be introduced inside the hydrous gel phase polymer. Thus, the suction power of superabsorbent polymer may be increased without degradation of other properties.

The blowing agent is not specifically limited as long as it can generate gas under the temperature condition applied when forming the hydrous gel phase polymer. For example, one or more selected from the group consisting of sodium bicarbonate(SBC), 4,4'-Oxybis(benzenesulfonyl hydrazide) (OBSH), p-toluenesulfonyl hydrazide (TSH), sugar ester and acetone may be used.

The blowing agent may be added in the concentration of about 0.001 to 1 wt%, based on the monomer composition.

Besides, the monomer composition may further comprise additives such as a thickener, a plasticizer, a preservation stabilizer, an antioxidant, and so on.

Further, the monomer composition may be prepared in the form of a solution wherein raw materials such as above explained monomers, a polymerization initiator, a first cross-linking agent, and so on, are dissolved in a solvent. Here, the solvent that can be used is not limited in terms of its construction as long as it can dissolve the above explained components. For example, as the solvent, water, ethanol, ethyleneglycol, diethyleneglycol, triethyleneglycol, 1,4-butanediol, propyleneglycol, ethyleneglycol monobutyl ether, propyleneglycol monomethyl ether, propyleneglycol monomethyl ether acetate, methylethylketone, acetone, methylamylketone, cyclohexanone, cyclopentanone, diethyleneglycol monomethyl ether, diethyleneglycol ethyl ether, toluene, xylene, butyrolactone, carbitol, methylcellosolve acetate and N,N-dimethylacetamide, or a mixture thereof may be used.

Further, the formation of hydrous gel phase polymer through the polymerization of the monomer composition may be conducted by common polymerization methods, and the process is not specifically limited. As non-limiting examples, the polymerization method is largely classified into thermal polymerization and photo-polymerization according to energy source, and commonly, thermal polymerization may be progressed in a reactor equipped with a stirring axis such as a kneader, and photo-polymerization may be progressed in a reactor equipped with a movable conveyer belt

For example, hydrous gel phase polymer may be obtained by introducing the monomer composition into a reactor equipped with a stirring axis such as a kneader, and supplying hot wind or heating the reactor, thereby progressing thermal polymerization. Here, the hydrous gel phase polymer discharged to the outlet of the reactor may be obtained in the form of a few centimeters to a few millimeters according to the shape of the stirring axis equipped in the reactor. Specifically, the size of obtained hydrous gel phase polymer may vary according to the concentration of the introduced monomer composition and the introduction speed, and so on, and commonly, hydrous gel phase polymer having a (weight average) particle diameter of 2 to 50 mm may be obtained

Further, in case photo-polymerization is progressed in a reactor equipped with a movable conveyer belt as explained above, hydrous gel phase polymer in the form of a sheet may be obtained. Here, the thickness of the polymer sheet may vary according to the concentration of the introduced monomer composition and the introduction speed, but, commonly, it is preferable that the thickness is controlled to 0.5 to 5 cm so as to secure the production speed while uniformly polymerizing the whole sheet.

The hydrous gel phase polymer obtained by such a method may exhibit a moisture content of about 40 to about 80 wt%. Here, the "moisture content" is the content of occupying moisture based on the total weight of hydrous gel phase polymer, and it means a value obtained by subtracting the weight of polymer of a dry state from the weight of hydrous gel phase polymer. Specifically, it is defined as a value calculated by measuring the weight loss according to moisture evaporation in the polymer while raising the temperature of polymer through infrared heating to dry. At this time, the drying condition is established such that the temperature is raised from room temperature to about 180°C and then maintained at 180°C, and the total drying time is 20 minutes including a temperature raising step of 5 minutes.

**Step of drying hydrous gel phase polymer (step 2)**

Meanwhile, the preparation method of superabsorbent polymer comprises a step of drying the hydrous gel phase polymer formed through the above explained step.

At this time, if necessary, in order to increase the efficiency of the drying step, a step of coarse crushing may be conducted before the drying.

As non-limiting examples, crushers that can be used in the coarse crushing include a vertical pulverizer, a turbo cutter, a turbo grinder, a rotary cutter mill, a cutter mill, a disc mill, a shred crusher, a crusher, a chopper, a disc cutter, and so on.

The coarse crushing step may be progressed such that the particle diameter of hydrous gel phase polymer may become 2 to 10 mm. That is, in order to increase drying efficiency, it is preferable that the hydrous gel phase polymer is crushed to particles of 10 mm or less. However, since particles may be agglomerated when excessively crushed, it is preferable that the hydrous gel phase polymer is crushed to particles of 2 mm or more.

Further, in case the coarse crushing step is conducted before the step of drying hydrous gel phase polymer, since the polymer is in the state of high moisture content, the polymer may adhere to the surface of the crusher. In order to minimize this phenomenon, in the coarse crushing step, an agent for preventing particle agglomeration such as steam, water, surfactant, clay or silica, and so on; a thermal polymerization initiator such as a persulfate-based initiator, an azo-based initiator, hydrogen peroxide, and ascorbic acid; an epoxy-based cross-linking agent, a diol cross-linking agent, a cross-linking agent comprising multifunctional acrylate such as difunctional or trifunctional or more, a monofunctional compound comprising a hydroxyl group may be added, as necessary.

Meanwhile, the drying of hydrous gel phase polymer immediately after coarse crushing or polymerization may be carried out at a temperature of 120 to 250°C, or 150 to 200°C, or 160 to 180°C (wherein, the temperature may be defined as the temperature of heat medium supplied for drying or the temperature inside a drying reactor including heat medium and polymer in the drying process.). That is, if the drying temperature is low and the drying time lengthens, the properties of the final polymer may be degraded, and in order to prevent this, it is preferable that the drying temperature is 120°C or more. Further, if the drying temperature is higher than is necessary, only the surface of hydrous gel phase polymer may be dried, thus generating a lot of fine particles in the grinding process as described below, and the properties of the final polymer may be degraded, and in order to prevent this, it is preferable that the drying temperature is 250°C or less.

Wherein, the drying time is not specifically limited, but considering process efficiency, and so on, it may be controlled to 20 to 90 minutes under the above drying temperature.

Further, the drying method is not limited in terms of the construction as long as it can be commonly used as a drying process of hydrous gel phase polymer. Specifically, in the drying step, hot wind supply, infrared ray irradiation, ultrahigh frequency wave irradiation, or UV irradiation, and so on, may be applied.

The polymer dried by such a method may exhibit a moisture content of about 0.1 to 10 wt%. That is, if the moisture content of the polymer is less than 0.1 wt%, due to excessive drying, a production cost may increase and the cross-linked polymer may be degraded, which is not favorable. Further, if the moisture content of the polymer exceeds 10 wt%, faulty may be generated in the subsequent process, which is not preferable.

**Step of grinding dried polymer (step 3)**

A step of grinding the polymer dried through the above explained step is conducted. The grinding step is intended to optimize the surface area of dried polymer, and it may be conducted such that the particle diameter of ground polymer may become 150 to 850 um.

At this time, as a grinder, a pin mill, a hammer mill, a screw mill, a roll mill, a disc mill, a jog mill, and so on, may be used. Further, in order to manage the properties of the finally productized superabsorbent polymer, a step of selectively sieving particles having a particle diameter of 150 to 850 um in the polymer particles obtained through the grinding step may be further conducted.

**Step of surface cross-linking ground polymer (step 4)**

A step of surface cross-linking the polymer ground through the above explained step by a second cross-linking agent is conducted.

The surface cross-linking is a step of inducing a cross-linking reaction on the surface of the ground polymer in the presence of a second cross-linking agent (surface cross-linking agent), thereby forming superabsorbent polymer having more improved properties. Through the surface cross-linking, a surface cross-linking layer is formed on the surface of the ground polymer particles.

The surface modification may be conducted by a common method of increasing the cross-linking density of a particle surface, and for example, it may be conducted by mixing a solution comprising a second cross-linking agent (surface cross-linking agent) with the ground polymer, and cross-linking it.

Here, the second cross-linking agent is a compound capable of reacting with the functional group of the polymer, and the construction is not specifically limited. However, as non-limiting examples, the second cross-linking agent may be one or more compound selected from the group consisting of ethylene glycol diglycidyl ether, polyethylene glycol diglycidyl ether, glycerol polyglycidyl ether, propylene glycol diglycidyl ether, polypropylene glycol diglycidyl ether, ethylene glycol, diethylene glycol, propylene glycol, triethylene glycol, tetraethylene glycol, propanediol, dipropylene glycol, polypropylene glycol, glycerin, polyglycerin, butanediol, heptanediol, hexanediol, trimethylol propane, pentaerythritol, sorbitol, calcium hydroxide, magnesium hydroxide, aluminum hydroxide, ferrous hydroxide, calcium chloride, magnesium chloride, aluminum chloride, and ferrous chloride.

Wherein, the content of the second cross-linking agent may be appropriately controlled according to the kind of the cross-linking agent or reaction conditions, and so on, and preferably, it may be controlled to 0.001 to 5 parts by weight, based on 100 parts by weight of the ground polymer. If the content of the second cross-linking agent is too low, surface cross-linking may not be properly achieved, and thus, the properties of the final polymer may be degraded. To the contrary, if an excessive amount of the second cross-linking agent is used, due to the excessive surface cross-linking reaction, the absorption power of the polymer may be rather decreased, which is not preferable.

Meanwhile, the surface cross-linking step may be conducted by common methods such as introducing the second cross-linking agent and the ground polymer in a reactor and mixing them, spraying the second cross-linking agent to the ground polymer, continuously supplying the ground polymer and the second cross-linking agent into a continuously operated mixer and mixing them, and so on.

Further, when the second cross-linking agent is added, water may be additionally added. As such, by adding the second cross-linking agent and water together, uniform dispersion of the second cross-linking agent may be induced, agglomeration of polymer particles may be prevented, and the penetration depth of the second cross-linking agent into polymer particles may be more optimized. Considering these objects and effects, the content of water added together with the second cross-linking agent may be controlled to 0.5 to 10 parts by weight, based on 100 parts by weight of the ground polymer.

Meanwhile, the present invention is characterized in that the surface cross-linking is carried out at 180 to 250°C. Since internal pores were previously formed by a blowing agent in step 1, on the surface of superabsorbent polymer, the surface area is also widened by the internal pores, and thus, it is preferable that the surface cross-linking is carried out at high temperature to increase surface cross-linking density. More preferably, the surface cross-linking is carried out at 190°C or more, and 240°C or less, 230°C or less, 220°C or less, 210°C or less, or 200°C or less.

Further, the surface cross-linking reaction may be progressed for 50 minutes or more. That is, in order to induce minimum surface cross-linking reaction and prevent damage of polymer particles and property degradation due to the excessive reaction, it may be progressed under the above explained surface cross-linking reaction conditions. Further, the reaction may be progressed for 120 minutes or less, 100 minutes or less, or 60 minutes or less.

**Superabsorbent polymer**

The superabsorbent polymer prepared by the preparation method of the present invention is characterized by increased suction power.

Specifically, the superabsorbent polymer according to the present invention has suction power of 15.0 mL/g or more. The suction power can be measured as described in Experimental Examples below. Preferably, the suction power is 15.5 mL/g or more, 16.0 mL/g or more, 16.5 mL/g or more, or 17.0 mL/g or more. As the suction power is higher, the properties of the superabsorbent is more excellent, and although the upper limit is not limited, for example, it may be 18.0 mL/g or less, 17.9 mL/g or less, 17.8 mL/g or less, 17.7 mL/g or less, 17.6 mL/g or less, or 17.5 mL/g or less.

Further, the superabsorbent polymer according to the present invention has excellent gel bed permeability as well as suction power. The gel bed permeability can be measured as described in Experimental Examples below. Preferably, the gel bed permeability is 30 darcy or more. Preferably, the gel bed permeability is 31 darcy or more, 32 darcy or more, 33 darcy or more, 34 darcy or more, or 35 darcy or more. Further, as the gel bed permeability is higher, the property of the superabsorbent polymer is more excellent, and although the upper limit is not limited, for example, it may be 150 darcy or less, 140 darcy or less, 130 darcy or less, 120 darcy or less, 110 darcy or less, 100 darcy or less, 90 darcy or less, 80 darcy or less, 70 darcy or less, or 60 darcy or less.

Further, the superabsorbent polymer according to the present invention has excellent absorbency under load as well as suction power. The absorbency under load can be measured as described in Experimental Examples below. Preferably, the absorbency under load may be 18.5 g/g or more. Preferably, the absorbency under load may be 19.0 g/g or more, 19.5 g/g or more, or 20.0 g/g or more. Further, as the absorbency under load is higher, the property of the superabsorbent polymer is more excellent, and although the upper limit is not limited, for example, it may be 25 g/g or less, 24 g/g or less, or 23 g/g or less.

Further, the superabsorbent polymer according to the present invention has excellent centrifuge retention capacity (CRC) as well as suction power. The centrifuge retention capacity can be measured as described in Experimental Examples below. Preferably, the centrifuge retention capacity may be 28.0 g/g or more. Preferably, the centrifuge retention capacity may be 28.5 g/g or more , 29.0 g/g or more, 29.5 g/g or more, 30.0 g/g or more, 30.5 g/g or more, or 31.0 g/g or more. Further, as the absorbency under load is higher, the property of the superabsorbent polymer is more excellent, and although the upper limit is not limited, for example, it may be 40 g/g or less, 39 g/g or less, 38 g/g or less, 37 g/g or less, 36 g/g or less, or 35 g/g or less.

Further, the superabsorbent polymer according to the present invention has excellent vortex as well as suction power. The vortex can be measured as described in Experimental Examples below. Preferably, the vortex is 60 s or less. Preferably, the vortex is 55 s or less, 50 s or less, 45 s or less, or 40 s or less.

As explained above, the superabsorbent polymer according to the present invention has excellent suction power without degradation of other properties required in superabsorbent polymer.

**[ADVANTAGEOUS EFFECTS]**

The preparation method of superabsorbent polymer according to the present invention can increase suction power without degradation of other properties of superabsorbent polymer, and thus, the prepared superabsorbent polymer may be usefully used as material of hygienic goods such as a diaper.

**[BRIEF DESCRIPTION OF THE DRAWINGS]**

Fig. 1 is a schematic diagram showing one example of an apparatus for measuring gel bed permeability (GBP) according to one embodiment of the present invention, and Fig. 2 and Fig. 3 are schematic diagrams respectively showing one example of a gel bed permeability measuring cylinder and mesh arrangement.
Fig.4 shows an apparatus for measuring suction power.

**[DETAILED DESCRIPTION OF THE EMBODIMENTS]**

Hereinafter, preferable examples are presented for a better understanding of the invention. However, these examples are presented only to illustrate the present invention, and the scope of the invention is not limited thereto.

**Comparative Example 1**

A partially neutralized acrylic acid aqueous solution was mixed with 2300 ppm of polyethyleneglycol diacrylate (PEGDA) as an internal cross-linking agent, 1500 ppm of potassium persulfate ($K_2S_2O_8$ as a thermal polymerization initiator, 100 ppm of hydrogen peroxide and 100 ppm of ascorbic acid, and thermal polymerization was progressed to obtain a polymerized sheet. The polymerized sheet was taken out and cut to a size of 3 cm x 3 cm, followed by chopping with a meat copper to prepare crumb. The crumb was dried in an oven capable of transferring air volume upward and downward. Hot air of 180°C was flowed from the lower part to the upper part for 15 minutes, and from the upper part to the lower part for 15 minutes, thus uniformly drying, and drying was conducted such that the moisture content after drying became 2% or less. After drying, grinding was conducted with a grinder, followed by sieving to select those with particle diameters of 150 to 850 um, thus preparing a base resin. Thereafter, based on 100 g of the base resin, 4 g of water, 3.5 g of methanol, 0.15 g of ethyleneglycol diglycidyl ether, 0.10 g of Aerosil 200 were added and mixed, and then, reacted at a surface cross-linking temperature of 195°C for 1 hour, and ground to obtain surface treated superabsorbent polymer with a particle diameter of 150 to 850 um using a sieve.

**Comparative Example 2**

Superabsorbent polymer was prepared by the same method as Comparative Example 1, except that 4500 ppm of polyethyleneglycol diacrylate (PEGDA) was used as the internal cross-linking agent.

**Example 1**

Superabsorbent polymer was prepared by the same method as Comparative Example 1, except that 1000 ppm of SBC was used as a blowing agent.

**Example 2**

Superabsorbent polymer was prepared by the same method as Comparative Example 2, except that 1000 ppm of SBC was used as a blowing agent.

**Comparative Example 3**

Superabsorbent polymer was prepared by the same method as Comparative Example 1, except that the surface cross-linking temperature was 140°C.

**Comparative Example 4**

Superabsorbent polymer was prepared by the same method as Example 1, except that the surface cross-linking temperature was 140°C.

**Comparative Example 5**

Superabsorbent polymer was prepared by the same method as Comparative Example 2, except that the surface cross-linking temperature was 140°C.

**Comparative Example 6**

Superabsorbent polymer was prepared by the same method as Example 2, except that the surface cross-linking temperature was 140°C.

**Experimental Example**

For the superabsorbent polymer prepared in Examples and Comparative Examples, properties were evaluated as follows.

**(1) Gel Bed Permeability (GBP)**

For the superabsorbent polymer prepared in Examples and Comparative Examples, gel bed permeability (GBP) was measured. The measurement method of GBP is specified in US Patent No.7,179,851.

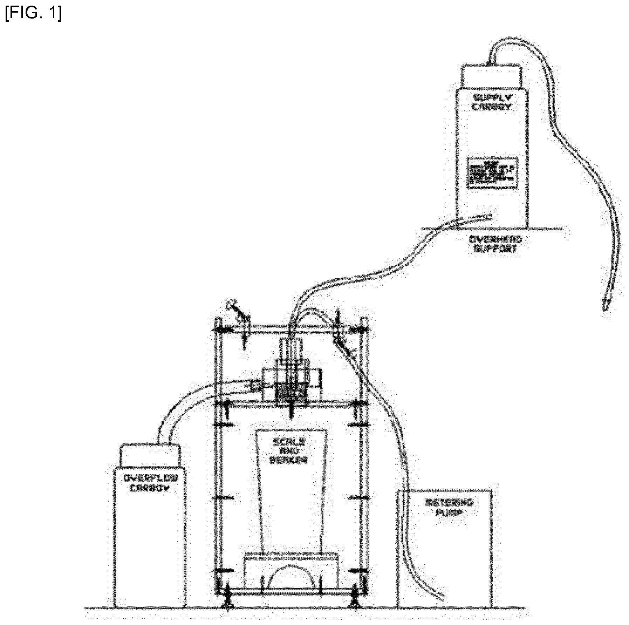

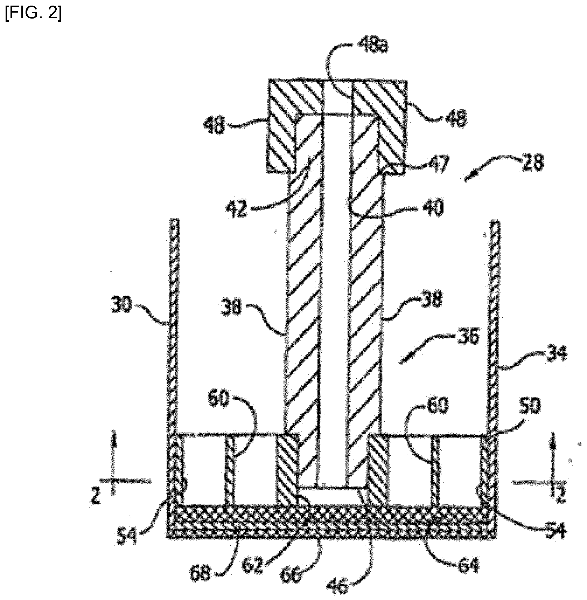

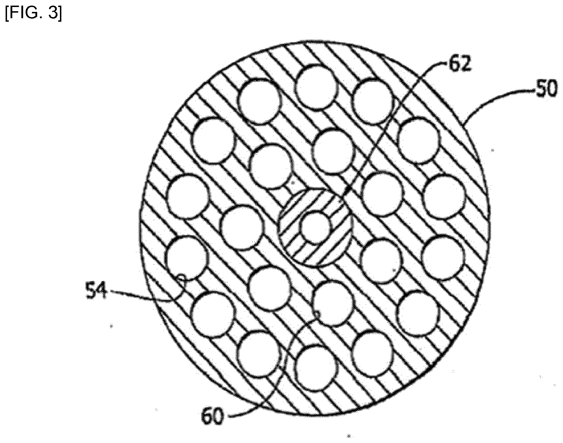

First, the apparatus suitable for conducting a gel bed permeability test is shown in Fig. 1, and specifically shown in Fig. 2 and Fig. 3. The test apparatus (28) comprises a sample container(generally indicated as 30) and a piston(generally indicated as 35). The piston (35) comprises a cylindrical LEXANR shaft (38) having a concentric cylindrical hole (40) bored down the longitudinal axis of the shaft. Both ends of the shaft (38) are machined to provide upper and lower ends (respectively designated as 42 and 46). A weight (indicated as 48) rests on one end (42) and has a cylindrical hole (48a) bored through at least a portion of its center.

A circular piston head (50) is positioned on the other end (46) and is provided with a concentric inner ring of seven holes (60, each having a diameter of about 0.95 cm), and a concentric outer ring of fourteen holes (54, also each having a diameter of about 0.95 cm). The holes (54,60) are bored from the top to the bottom of the piston head (50). The piston head (50) also has a cylindrical hole (62) bored in the center thereof to receive end (46) of the shaft (38). The bottom of the piston head (50) may also be covered with a biaxially stretched 400 mesh stainless steel screen (64).

The sample container (30) comprises a cylinder (34) and a 400 mesh stainless steel cloth screen (66) that is biaxially stretched to tautness and attached to the lower end of the cylinder. A superabsorbent polymer sample (indicated as 68 in Fig. 2) is supported on the screen (66) within the cylinder (34) during testing.

The cylinder (34) may be bored from a transparent LEXAN rod or equivalent material, or it may be cut from a LEXAN tubing or equivalent material, and has an inner diameter of about 6 cm (e.g., a cross-sectional area of about 28.27 cm$^2$), a wall thickness of about 0.5 cm and a height of approximately 10 cm. Drainage holes (not shown) are formed in the sidewall of the cylinder (34) at a height of approximately 7.8 cm above the screen (66) to allow liquid to drain from the cylinder to thereby maintain a fluid level in the sample container at approximately 7.8 cm above the screen (66). The piston head (50) is machined from a LEXAN rod or equivalent material and has a height of approximately 16 mm and a diameter sized such that it fits within the cylinder (34) with minimum wall clearance but still slides freely. The shaft (38) is machined from a LEXAN rod or equivalent material and has an outer diameter of about 2.22 cm and an inner diameter of about 0.64 cm.

The shaft upper end (42) is approximately 2.54 cm long and approximately 1.58 cm in diameter, forming an annular shoulder (47) to support the weight (48). The annular weight (48) has an inner diameter of about 1.59 cm so that it slips onto the upper end (42) of the shaft (38) and rests on the annular shoulder (47) formed thereon. The annular weight (48) can be made from stainless steel or from other suitable materials resistant to corrosion in the presence of the test solution, which is 0.9 wt% sodium chloride solution in distilled water. The combined weight of the piston (35) and annular weight (48) equals approximately 596 g, which corresponds to a pressure applied to the sample (68) of about 0.3 psi, or about 20.7 g/cm$^2$, over a sample area of about 28.27 cm$^2$.

When the test solution flows through the test apparatus during testing as described below, the sample container (30) generally rests on a 16 mesh rigid stainless steel support screen (not shown). Alternatively, the sample container (30) may rest on a support ring (not shown) diametrically sized substantially the same as the cylinder (34) so that the support ring does not restrict flow from the bottom of the container.

To conduct the Gel Bed Permeability Test under "free swell" conditions, the piston (35), with the weight (48) seated thereon, is placed in an empty sample container (30) and the height from the bottom of the weight (48) to the top of the cylinder (34) is measured using a caliper accurate to 0.01 mm. It is important to measure the height of each sample container (30) empty and to keep track of which piston (35) and weight (48) is used when using multiple test apparatus. The same piston (35) and weight (48) should be used for measurement when the sample (68) is later swollen following saturation.

The sample to be tested is prepared from superabsorbent material particles which are prescreened through a U.S. standard 30 mesh screen and retained on a U.S. standard 50 mesh screen. As a result, the test sample comprises particles sized in the range of about 300 to about 600 um. The particles can be prescreened by hand or automatically. Approximately 2.0 g of the sample is placed in the sample container (30), and the container, without the piston (35) and weight (48) therein, is then submerged in the test solution for a time period of about 60 minutes to saturate the sample and allow the sample to swell free of any restraining load.

At the end of this period, the piston (35) and weight (48) assembly is placed on the saturated sample (68) in the sample container (30) and then the sample container (30), piston (35), weight (48), and sample (68) are removed from the solution. The thickness of the saturated sample (68) is determined by again measuring the height from the bottom of the weight (48) to the top of the cylinder (34), using the same thickness clipper or measuring instrument used previously (provided that the zero point is unchanged from the initial height measurement). The height measurement obtained from measuring the empty sample container (30), piston (35), and weight (48) is subtracted from the height measurement obtained after saturating the sample (68). The resulting value is the thickness, or height "H" of the swollen sample.

The permeability measurement is initiated by delivering a flow of the test solution into the sample container (30) with the saturated sample (68), piston (35), and weight (48) inside. The flow rate of the test solution into the container is adjusted to maintain a fluid height of about 7.8 cm above the bottom of the sample container. The quantity of solution passing through the sample (68) versus time is measured gravimetrically. Data points are collected every second for at least twenty seconds once the fluid level has been stabilized to and maintained at about 7.8 cm in height. The flow rate Q through the swollen sample (68) is determined in units of grams/second (g/s) by a linear least-square fit of fluid passing through the sample (68) (in grams) versus time (in seconds).

Permeability (darcy) is obtained by the following equation:

[Equation 1]

$$K = [Q \times H \times Mu]/[A \times Rho \times P]$$

where K is permeability ($cm^2$), Q is flow rate (g/sec), H is height of sample (cm), Mu is liquid viscosity (poise) (approximately one centipoises for the test solution used with this Test), A is cross-sectional area for liquid flow ($cm^2$), Rho is liquid density ($g/cm^3$) (for the test solution used with this Test) and P is hydrostatic pressure (dynes/$cm^2$) (normally approximately 3,923 dynes/$cm^2$). The hydrostatic pressure is calculated from the following Equation 2.

[Equation 2]

$$P = Rho \times g \times h$$

where Rho is liquid density ($g/cm^3$), g is gravitational acceleration, nominally 981 cm/$sec^2$, and h is fluid height, e.g., 7.8 cm for the Gel Bed Permeability Test described herein.

**(2) Absorbency under Load (AUL)**

For the superabsorbent polymer prepared in Examples and Comparative Examples, Absorbency under Load (AUL) of 0.9 psi was measured as follows.

First, on the bottom of plastic cylinder of 25 mm inner diameter, stainless 400 mesh wire netting was installed. Superabsorbent polymer $W_0$ (g, 0.16 g) was uniformly sprayed onto the wire netting under room temperature and 50% humidity, and a piston capable of uniformly giving 5.1 kPa (0.9 psi) load thereon has an outer diameter slightly less than 25 mm and does not have a gap with the inner wall of cylinder, and the upward and downward movement is not hindered. Wherein, the weight $W_3$(g) of the apparatus was measured.

In the inner side of a petri dish of 150 mm diameter, a glass filter of 90 mm diameter and 5 mm thickness was placed, and a saline solution consisting of 0.90 wt% sodium chloride was placed to the same level as the upper side of the glass filter. One filter paper of 90 mm diameter was loaded thereon. The measuring apparatus was loaded on the filter paper, and the liquid was absorbed under load for 1 hour. After 1 hour, the measuring apparatus was lifted, and the weight $W_4$(g) was measured.

Using each weight obtained above, AUL (g/g) was calculated according to the following Equation 3.

[Equation 3]

$$AUL\ (g/g) = [W_4(g) - W_3(g)]/ W_0(g)$$

In the Equation 3,
$W_0$(g) is the weight (g) of absorbent polymer,
$W_3$(g) is the sum of the weight of absorbent polymer and the weight of the apparatus capable of giving a load to the polymer,
$W_4$(g) is the sum of the weight of moisture-absorbed absorbent polymer and the weight of the apparatus capable of giving a load to the absorbent polymer, after supplying moisture to the absorbent polymer under a load (0.9 psi) for 1 hour.

**(3) Suction Power (SP)**

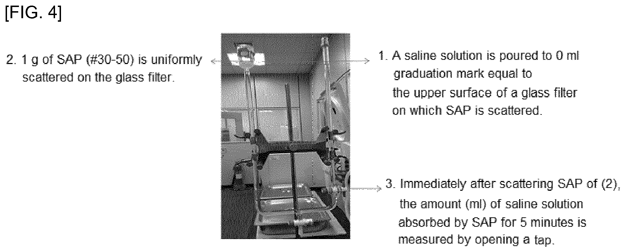

SP was measured with the measuring apparatus as shown in Fig. 4. Specifically, on the right side of the measuring apparatus, saline water (0.9% NaCl) was filled to the 0 mL gradation on a glass tube with 20 mm inner diameter. On the left side of the measuring apparatus, a 100 micrometer glass filter was installed on the bottom of a cylindrical funnel with 50 mm inner diameter, and 1.0 g of superabsorbent polymer was uniformly sprayed onto the glass filter under 23°C, 50% humidity conditions. Simultaneously with spraying superabsorbent polymer, the cock of a burette of the measuring apparatus was opened, and the amount of saline water (g) absorbed by 1g of superabsorbent polymer for 5 minutes was measured.

**(4) Centrifuge Retention Capacity (CRC)**

According to EDANA method WSP 241.2, centrifuge retention capacity of the polymer of Examples and Comparative Examples were measured. That is, the polymer W(g) (about 0.2 g) respectively obtained through Examples and Comparative Examples was uniformly put in an envelope made of non-woven fabrics and sealed, which is then submerged into a saline solution (0.9 wt%) at room temperature. After about 30 minutes elapsed, the envelope was drained for 3 minutes under 250G condition using a centrifuge, and the weight of the envelope $W_2$(g) was measured. Further, after the same operation without using polymer, the weight at that time $W_1$(g) was measured. Using each obtained weight, CRC (g/g) was calculated according to the following Equation.

[Equation 4]

$$CRC \ (g/g) = \{(W_2 - W_1) / W\} - 1$$

**(5) Vortex**

$50.0 \pm 1.0$ mL of a 0.9% NaCl solution was added to a 100 mL beaker. A cylindrical stirring bar (30 x 6 mm) was added, and the saline solution was stirred at 600 rpm on a stirring plate. $2.000 \pm 0.010$ g of superabsorbent polymer was added to the beaker as soon as possible, and at the beginning of the addition, a stopwatch was started. The stopwatch was stopped when the surface of the mixture became "still" state, which means that the surface does not have turbulent flow, at which time the mixture may still rotate but the whole surface of particles may rotate as one unit. The time indicated in the stopwatch was recorded as a vortex time.
The results are shown in the following Table 1.

[Table 1]

| | B/R[1] | | P/D[2] | | | | |
|---|---|---|---|---|---|---|---|
| | CRC (g/g) | Vortex (s) | CRC (g/g) | SP (mL/q) | AUL (g/g) | GBP (darcy) | Vortex (s) |
| Comparative Example 1 | 40.3 | 87 | 36.4 | 15.2 | 18.0 | 27 | 95 |
| Example 1 | 38.4 | 70 | 31.5 | 17.4 | 19.6 | 36 | 84 |
| Comparative Example 2 | 34.2 | 75 | 29.9 | 15.6 | 21.2 | 60 | 63 |
| Example 2 | 35.3 | 63 | 29.6 | 16.5 | 20.1 | 52 | 75 |
| Comparative Example 3 | 40.3 | 87 | 36.7 | 15.0 | 16.9 | 15 | 91 |
| Comparative Example 4 | 38.4 | 70 | 32.1 | 17.0 | 18.0 | 19 | 81 |
| Comparative Example 5 | 34.2 | 75 | 29.9 | 15.5 | 20.1 | 43 | 82 |
| Comparative Example 6 | 35.3 | 63 | 29.7 | 16.3 | 19.4 | 39 | 71 |
| 1) B/R: base resin before surface cross-linking | | | | | | | |
| 2) P/D: final superabsorbent polymer prepared in Example or Comparative Example | | | | | | | |

As shown in the Table 1, it was confirmed that the superabsorbent polymer prepared according to the present invention has remarkably increased suction power (SP) compared to Comparative Examples, and there is no significant difference or rather there is an improvement in the properties other than suction power.
Therefore, the preparation method of superabsorbent polymer according to the present invention is characterized by increasing suction power without degradation of other properties.

**Claims**

**1.** A method for preparing superabsorbent polymer comprising the steps of

1) polymerizing and cross-linking a monomer composition comprising a water-soluble ethylene-based unsaturated monomer, a polymerization initiator, a first cross-linking agent and a blowing agent to form a hydrous gel phase polymer,
2) drying the hydrous gel phase polymer,
3) grinding the dried polymer, and
4) carrying out a surface cross-linking reaction by reacting the ground polymer at 180 to 250°C for 50 minutes or more in the presence of a second cross-linking agent.

2. The method for preparing superabsorbent polymer according to claim 1, wherein the water-soluble ethylene-based unsaturated monomer is a compound represented by the following Chemical Formula 1,

[Chemical Formula 1] $R_1$-COOM$^1$

in the Chemical Formula 1,

$R_1$ is a $C_{2-5}$ alkyl group comprising an unsaturated bond,
$M^1$ is a hydrogen atom, a monovalent or divalent metal, an ammonium group, or an organic amine salt.

3. The method for preparing superabsorbent polymer according to claim 1, wherein the first cross-linking agent is one or more selected from the group consisting of N,N'-methylenebisacrylamide, trimethylolpropane tri(meth)acrylate, ethyleneglycol di(meth)acrylate, polyethyleneglycol (meth)acrylate, propyleneglycol di(meth)acrylate, polypropyleneglycol (meth)acrylate, butanediol di(meth)acrylate, butyleneglycol di(meth)acrylate, diethyleneglycol di(meth)acrylate, hexanediol di(meth)acrylate, triethyleneglycol hexanediol di(meth)acrylate, triethyleneglycol di(meth)acrylate, tripropyleneglycol di(meth)acrylate, tetraethyleneglycol di(meth)acrylate, dipentaerythritol pentaacrylate, glycerin tri(meth)acrylate, pentaerythritol pentaacrylate, triarylamine, ethyleneglycol diglycidyl ether, propylene glycol, glycerin, and ethylene carbonate.

4. The method for preparing superabsorbent polymer according to claim 1, wherein the blowing agent is one or more selected from the group consisting of sodium bicarbonate, 4,4'-oxybis(benzenesulfonyl hydrazide), p-toluenesulfonyl hydrazine, sugar ester, and acetone.

5. The method for preparing superabsorbent polymer according to claim 1, further comprising the step of crushing the hydrous gel phase polymer to a particle diameter of 2 to 10 mm, before the step 2) of drying the hydrous gel phase polymer.

6. The method for preparing superabsorbent polymer according to claim 1, wherein the step 3) of grinding is carried out such that the particle diameter of the ground polymer becomes 150 to 850 um.

7. The method for preparing superabsorbent polymer according to claim 1, wherein the second cross-linking agent is one or more selected form the group consisting of ethylene glycol diglycidyl ether, polyethylene glycol diglycidyl ether, glycerol polyglycidyl ether, propylene glycol diglycidyl ether, polypropylene glycol diglycidyl ether, ethylene glycol, diethylene glycol, propylene glycol, triethylene glycol, tetraethylene glycol, propanediol, dipropylene glycol, polypropylene glycol, glycerin, polyglycerin, butanediol, heptanediol, hexanediol, trimethylol propane, pentaerythritol, sorbitol, calcium hydroxide, magnesium hydroxide, aluminum hydroxide, ferrous hydroxide, calcium chloride, magnesium chloride, aluminum chloride, and ferrous chloride.

8. The method for preparing superabsorbent polymer according to claim 1, wherein the surface cross-linking reaction is carried out at 180 to 200°C.

9. The method for preparing superabsorbent polymer according to claim 1, wherein the suction power of the superabsorbent polymer is 15.0 mL/g or more.

[FIG. 1]

[FIG. 2]

[FIG. 3]

[FIG. 4]

2. 1 g of SAP (#30-50) is uniformly scattered on the glass filter.

1. A saline solution is poured to 0 ml graduation mark equal to the upper surface of a glass filter on which SAP is scattered.

3. Immediately after scattering SAP of (2), the amount (ml) of saline solution absorbed by SAP for 5 minutes is measured by opening a tap.

## INTERNATIONAL SEARCH REPORT

International application No.

**PCT/KR2015/013676**

### A. CLASSIFICATION OF SUBJECT MATTER

*C08J 3/24(2006.01)i, C08J 3/075(2006.01)i, C08F 2/10(2006.01)i, C08J 9/10(2006.01)i, C08J 9/14(2006.01)i*

According to International Patent Classification (IPC) or to both national classification and IPC

### B. FIELDS SEARCHED

Minimum documentation searched (classification system followed by classification symbols)

C08J 3/24; C08F 2/10; C08J 3/075; C08F 220/06; C08F 2/44; C08F 220/14; A61L 15/24; C08F 20/06; C08J 9/10; C08J 9/14

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)

eKOMPASS (KIPO internal) & Keywords: ethylenically unsaturated monomer, first crosslinking agent, second crosslinking agent, foaming agent, polymerization initiator, dry, pulverization, surface crosslinking

### C. DOCUMENTS CONSIDERED TO BE RELEVANT

| Category* | Citation of document, with indication, where appropriate, of the relevant passages | Relevant to claim No. |
|---|---|---|
| X | KR 10-2015-0116418 A (EVONIK CORPORATION) 15 October 2015<br>See paragraphs [0070], [0073], [0076], [0081], [0114], [0118], [0119], [0142]; and claim 1. | 1-4,6-9 |
| Y | | 5 |
| Y | KR 10-2011-0134333 A (LG CHEM. LTD.) 14 December 2011<br>See paragraph [0054]; and claim 1. | 5 |
| A | KR 10-2014-0026506 A (EVONIK DEGUSSA GMBH.) 05 March 2014<br>See claims 1, 2. | 1-9 |
| A | KR 10-2014-0125420 A (NIPPON SHOKUBAI CO., LTD.) 28 October 2014<br>See claim 1. | 1-9 |
| A | KR 10-0331343 B1 (CHEMISCHE FABRIK STOCKHAUSEN GMBH.) 08 August 2002<br>See claim 1. | 1-9 |

☐ Further documents are listed in the continuation of Box C.      ☒ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 26 JULY 2016 (26.07.2016) | **29 JULY 2016 (29.07.2016)** |

| Name and mailing address of the ISA/KR | Authorized officer |
|---|---|
| Korean Intellectual Property Office<br>Government Complex-Daejeon, 189 Seonsa-ro, Daejeon 302-701, Republic of Korea | |
| Facsimile No. 82-42-472-7140 | Telephone No. |

Form PCT/ISA/210 (second sheet) (January 2015)

INTERNATIONAL SEARCH REPORT
Information on patent family members

International application No.

**PCT/KR2015/013676**

| Patent document cited in search report | Publication date | Patent family member | Publication date |
|---|---|---|---|
| KR 10-2015-0116418 A | 15/10/2015 | EP 2930191 A1<br>JP 2015-199958 A<br>US 2015-0283284 A1 | 14/10/2015<br>12/11/2015<br>08/10/2015 |
| KR 10-2011-0134333 A | 14/12/2011 | US 2011-0301303 A1<br>US 8507620 B2 | 08/12/2011<br>13/08/2013 |
| KR 10-2014-0026506 A | 05/03/2014 | CN 103476811 A<br>DE 102011007723 A1<br>EP 2699609 A1<br>JP 2014-516378 A<br>TW 201302876 A<br>TW I506065 B<br>US 2014-0054497 A1<br>WO 2012-143235 A1 | 25/12/2013<br>25/10/2012<br>26/02/2014<br>10/07/2014<br>16/01/2013<br>01/11/2015<br>27/02/2014<br>26/10/2012 |
| KR 10-2014-0125420 A | 28/10/2014 | CN 104136505 A<br>JP 05795813 B2<br>US 2015-0011388 A1<br>WO 2013-122246 A1 | 05/11/2014<br>14/10/2015<br>08/01/2015<br>22/08/2013 |
| KR 10-0331343 B1 | 08/08/2002 | AU 1999-33207 A1<br>EP 0707603 A1<br>EP 0707603 B1<br>JP 02849617 B2<br>JP 08-509521 A<br>KR 10-1996-0703952 A<br>US 5712316 A<br>WO 95-02002 A1 | 26/08/1999<br>02/05/1997<br>03/09/1997<br>20/01/1999<br>08/10/1996<br>31/08/1996<br>27/01/1998<br>19/01/1995 |

Form PCT/ISA/210 (patent family annex) (January 2015)

## REFERENCES CITED IN THE DESCRIPTION

### Patent documents cited in the description

- KR 1020150161157
- JP SHO56161408 B
- JP SHO57158209 B
- JP SHO57198714 B
- US 7179851 B

### Non-patent literature cited in the description

- **REINHOLD SCHWALM.** UV Coatings: Basics, Recent Developments and New Application. Elsevier, 2007, 115
- Principle of Polymerization. Wiley, 1981, 203